# FASCICULE DE BREVET EUROPEEN

(11) **EP 0 957 106 B1**
(45) Date de publication et mention de la délivrance du brevet: **12.03.2003**
(21) Numéro de dépôt: 99401143.5
(22) Date de dépôt: 10.05.1999
(51) Int. Cl.: C07H 3/04

(54) **Procédé de préparation de maltulose monohydrate cristallisé**
Verfahren zur Herstellung von Kristallin-Maltulose-Monohydrat
Process for the preparation of crystalline maltulose monohydrate

(30) Priorité: 12.05.1998 FR 9805951
(43) Date de publication de la demande: 17.11.1999
(73) Titulaire: Roquette Frères, 62136 Lestrem (FR)
(72) Inventeur: Fouache, Catherine, 62113 Sailly Labourse (FR); Choque, Jean-Christophe, 62400 Bethune (FR)
(74) Mandataire: Boulinguiez, Didier

(56) Documents cités:
- FR-A- 2 055 645
- P.E.PFEFFER ET AL.: "Solid-State Structures of Keto-Disaccharides as Probed by 13C Cross-Polarization, "Magic-Angle" Spinning N.M.R. Spectroscopy." CARBOHYDRATE RESEARCH, vol. 111, 1983, pages 181-194, XP002091737 Amsterdam, NL
- L.HOUGH ET AL.: "The Reaction of Amino-Compounds with Sugars. Part II. The Action of Ammonia on Glucose, Maltose and Lactose." JOURNAL OF THE CHEMICAL SOCIETY. PART II., 1953, pages 2005-2009, XP002091738 LETCHWORTH GB

## Description

La présente invention concerne un procédé de préparation de cristaux de maltulose monohydrate par cristallisation du maltulose en solution aqueuse.

Le maltulose (4-O-α-D-glucopyranosyl-D-fructose) n'est pas un produit largement répandu dans la nature. On en trouve cependant dans le miel où il est synthétisé par réaction de transglucosylation durant l'hydrolyse du sucrose par les D-glucosidases et également dans les levures, où il est produit par un mécanisme enzymatique équivalent. Le maltulose est également détecté dans les hydrolysats d'amidon de maïs *waxy*, et dans les hydrolysats d'α-amylolyse du glycogène de foie de rat.

Le maltulose possède un fort pouvoir sucrant, équivalent à celui du maltitol, inférieur à celui du saccharose mais supérieur à celui du dextrose et du maltose. Le maltulose est utilisé sous forme de sirops dans les industries alimentaire et pharmaceutique, et comme matériaux de départ de diverses synthèses industrielles.

La préparation industrielle d'un sirop de maltulose repose sur l'isomérisation d'un sirop de maltose en milieu alcalin (HOUGH et al., 1953, *J. Chem. Soc.,* *Part* II, 2005; ROBERTS *et al*, *Biochem. J*., 1952, 51, XVII). Pour illustrer un tel procédé, on peut citer les travaux de HODGE *et al* (*Cereal Science Today,* 1972, 17, 180), qui décrivent la préparation d'un sirop de maltulose par isomérisation chimique d'un sirop de maltose en présence d'aluminate de sodium avec des rendements de conversion de l'ordre de 95%.

Des procédés permettant d'obtenir des sirops de maltulose par isomérisation d'hydrolysats d'amidon enrichis en maltose sont également décrits dans les brevets FR 2 055 645 et Re. 30 820.

En ce qui concerne l'obtention des cristaux purs de maltulose à partir de ces sirops, deux procédés sont connus, à savoir :
- celui décrit par HOUGH *et al.* dans *J. Chem. Soc.,* 1953, Part II, 2005, qui concerne la cristallisation du maltulose monohydrate dans le mélange dioxane-méthanol, conduisant à un composé de point de fusion (mp) compris entre 113 et 115°C ;
- celui décrit par PFEFFER et HICKS dans *Carb. Research.,* 1983, 111, 181, décrivant la cristallisation du maltulose monohydrate dans l'acétone. Les cristaux, lavés à l'acétone et séchés à l'air, possèdent un mp de 116,5 à 118°C.

Il apparait donc clairement dans l'état de la technique que les procédés de cristallisation du maltulose nécessitent la mise en oeuvre de solvants organiques toxiques et inflammables (dioxane-méthanol, acétone) pour obtenir des cristaux de maltulose. Or, l'utilisation de tels solvants présente un certain risque pour les manipulateurs, voire pour l'environnement.

Surtout, ces procédés rendent obligatoire la purification poussée du maltulose cristallisé pour éliminer toutes traces de solvants organiques. En effet, ces traces de solvant présenteraient une toxicité certaine dans le cadre des applications alimentaires et pharmaceutiques du maltulose.

Les procédés précédemment décrits ne donnent donc pas satisfaction car ils souffrent de l'inconvénient de ne pouvoir offrir une méthode simple de production de cristaux de maltulose sans traces de composés toxiques résultant de l'étape de cristallisation. Par conséquent, le maltulose cristallisé obtenu selon les procédés connus ne peut pas être directement utilisé en applications pharmaceutiques, par exemple pour la fabrication de poudres ou de comprimés.

L'invention a donc pour but de remédier à cette situation, et de proposer un procédé répondant mieux que ceux qui existent déjà aux diverses contraintes de la pratique.

En effet, la société Demanderesse a réussi à mettre au point un procédé industriel permettant d'obtenir du maltulose cristallisé à partir d'une solution aqueuse, et par conséquent exempt de tous composés toxiques.

Se faisant, la société Demanderesse a vaincu un préjugé technique à l'encontre de la mise en oeuvre d'un tel procédé faisant appel à l'eau en tant que solvant pour cristalliser le maltulose, car il n'a jamais été suggéré que l'on puisse cristalliser directement et facilement du maltulose dans l'eau et encore moins d'en isoler les cristaux ainsi formés.

Au contraire, en raison de la forte solubilité du maltulose dans l'eau, les spécialistes dans ce domaine ont conclu que la cristallisation du maltulose à partir d'une solution aqueuse était difficilement réalisable industriellement. Ainsi, dans le brevet HAYASHIBARA (FR 2 055 645) il est rappelé que le maltulose est très soluble dans l'eau et il est en outre affirmé que les sirops de maltulose sont difficilement cristallisables, même en solutions très concentrées, à la température ordinaire. Dans les conditions décrites dans ce brevet, le maltulose lorsqu'il cristallise, le fait sous forme de particules si fines et si mal formées qu'elles sont en tout cas inséparables du sirop.

Cette cristallisation présente en fait un certain nombre de difficultés techniques, que la société Demanderesse a résolu au terme de longues recherches.

L'objet de la présente invention est donc de proposer un procédé de préparation de cristaux de maltulose dénué des inconvénients précités.

Le procédé de préparation de maltulose monohydrate cristallisé est caractérisé par le fait qu'il comporte les étapes suivantes :
- préparation d'une solution aqueuse de maltulose d'une richesse supérieure à 65% en poids,
- concentration de la solution aqueuse de maltulose à une matière sèche supérieure à 50 % en poids et à une température telle que le degré de sursaturation du maltulose soit inférieur à 1,
- refroidissement de ladite solution concentrée de manière à porter le degré de sursaturation du maltulose à une valeur supérieure à 1,
- cristallisation du maltulose dans ladite solution sursaturée, en la refroidissant à vitesse contrôlée et sous agitation, et
- séparation, récupération et séchage des cristaux de maltulose monohydrate ainsi obtenus.

Le procédé selon l'invention comporte donc une première étape de préparation d'une solution aqueuse de maltulose de richesse supérieure à 65 % en poids. Les solutions les plus riches donneront bien évidemment les rendements de cristallisation les meilleurs.

L'enrichissement de la solution de maltulose pourra être réalisé par toutes méthodes en elles-mêmes connues, telles que par exemple les méthodes chromatographiques. De préférence, cet enrichissement sera mené de manière à obtenir une richesse en maltulose supérieure à 85 % en poids, et de manière plus préférentielle comprise entre 85 et 97 % en poids.

Selon un mode préférentiel de réalisation de l'invention, la solution aqueuse de maltulose est préparée par isomérisation en conditions alcalines d'un sirop riche en maltose.

Par exemple, on réalise l'isomérisation d'un sirop riche en maltose par ajoût d'acide borique en quantité au moins équimolaire. Le pH est alors ajusté à une valeur supérieure à 10, de préférence voisine de 11. L'isomérisation est ensuite conduite à une température de l'ordre de 50°C, pendant un délai compris en 4 et 5 heures. A l'issue de cette réaction d'isomérisation, le rendement de conversion du maltose en maltulose est supérieur à 80 %.

La solution de maltulose ainsi obtenue est alors refroidie à une température de l'ordre de 30°C, et son pH ajusté à 2 par ajoût d'acide chlorydrique.

On réalise ensuite l'élimination de l'acide borique résiduel. On préfère effectuer plusieurs jets de cristallisation de l'acide borique. Par exemple, deux jets de cristallisation permettent d'éliminer environ 80 % de l'acide borique initial.

On effectue alors sur cette solution de maltulose un tamisage moléculaire. Cette étape de tamisage moléculaire peut consister, par exemple, en une étape de séparation chromatographique ou en une étape de séparation sur membranes.

L'étape chromatographique est effectuée de manière connue en soi, de façon discontinue ou continue (lit mobile simulé), sur des adsorbants de type résines cationiques fortes à l'aide d'ions alcalins ou alcalino-terreux, tels que le calcium ou le magnésium, mais plus préférentiellement à l'aide d'ions sodium et dont le support est constitué de divinyl benzylidène à 4 %. La solution de maltulose ainsi obtenue est ensuite déminéralisée.

La seconde étape du procédé conforme à l'invention consiste à concentrer ladite solution aqueuse de maltulose pour obtenir une solution dont le taux de matière sèche est supérieur à 50 % en poids, à une température telle que le degré de sursaturation du maltulose soit inférieur à 1.

Selon un mode de réalisation préférentielle de l'invention, la solution de maltulose obtenue à l'issue de la seconde étape du procédé présente un taux de matière sèche compris entre 65 et 75 % en poids. Ce taux pourra être d'autant plus faible que la richesse en maltulose de la solution sera élevée.

Cette étape de concentration est effectuée de manière en elle-même connue, par exemple par évaporation de l'eau sous vide à une température supérieure à 60°C, de manière à obtenir une solution concentrée dont le degré de sursaturation du maltulose soit inférieur à 1.

Une caractéristique importante du procédé selon l'invention est qu'au terme de cette seconde étape, on obtient une solution aqueuse concentrée en maltulose à une température telle que son degré de sursaturation en maltulose est d'une valeur inférieure à 1. Cette température sera préférentiellement comprise entre 60°C et 80°C pour des solutions aqueuses de maltulose d'une richesse supérieure à 65 % en poids et d'un taux de matière sèche supérieur à 50 % en poids.

La troisième étape du procédé conforme à l'invention consiste alors à refroidir ladite solution de manière à porter le degré de sursaturation du maltulose à une valeur supérieure à 1.

Un degré de sursaturation en maltulose légèrement supérieur à 1, soit compris entre 1 et 1,2, plus préférentiellement compris entre 1,05 et 1,15 est atteint en refroidissant la solution aqueuse de maltulose concentrée, obtenue à l'issue de la deuxième étape du procédé conforme à l'invention, à une valeur qui sera fonction de sa teneur initiale en maltulose et de son taux de matière sèche.

Par exemple, on refroidit les solutions aqueuses de maltulose d'une richesse supérieure à 65 % en poids et d'un taux de matière sèche supérieur à 50 % en poids à des températures comprises généralement entre 20°C et 50°C.

Plus particulièrement, on refroidit les solutions aqueuses de maltulose d'une richesse comprise entre 85 et 97 % en poids et d'un taux de matière sèche compris entre 65 et 75 % en poids à des températures comprises entre 25°C et 35°C.

La quatrième étape du procédé conforme à l'invention, consiste alors à cristalliser le maltulose dans ladite solution aqueuse sursaturée, en la refroidissant à vitesse contrôlée et sous agitation.

Les autres recherches effectuées par la société Demanderesse pour cristalliser le maltulose dans l'eau ont en effet montré que les autres procédés de cristallisation consistant à faire varier le degré de sursaturation du maltulose par des techniques autres que l'abaissement de la température, par exemple par évaporation de l'eau tel que pratiqué dans un procédé d'évapocristallisation, ne donnaient pas entière satisfaction.

La société Demanderesse a donc eu le mérite de montrer que la solubilité du maltulose diminue notablement avec l'abaissement de la température et que le degré de sursaturation en maltulose se maintient à une valeur supérieure à 1 quand on refroidit lentement la solution aqueuse de maltulose obtenue au terme de la troisième étape.

La quatrième étape du procédé selon l'invention peut être initiée par la formation de germes de cristallisation, que l'on obtiendra par nucléation spontanée ou par ensemencement de ladite solution aqueuse de maltulose.

Selon un premier mode préférentiel de réalisation de l'invention, on choisit d'abaisser la température de la solution aqueuse de maltulose obtenue à l'issue de la troisième étape et de provoquer une nucléation spontanée par cisaillement de la solution.

Selon un second mode préférentiel de réalisation de l'invention, on choisit d'abaisser la température de la solution aqueuse de maltulose obtenue à l'issue de la troisième étape et de l'ensemencer avec des cristaux de maltulose.

Il est connu de l'homme de métier, de façon générale, que la vitesse de croissance et la taille des cristaux obtenus peuvent varier considérablement avec la vitesse d'abaissement de la température.

Les recherches effectuées par la société Demanderesse ont conduit celle-ci à établir un profil de diminution de la température, qui provoque la croissance des cristaux de maltulose de manière tout à fait satisfaisante.

D'une manière générale, ladite solution de maltulose est refroidie d'une température au plus égale à 50°C, préférentiellement d'une température comprise entre 25°C et 35°C, à une température d'au plus de 10°C, dans un délai d'au moins 15 heures.

Selon un mode préférentiel de réalisation du procédé conforme à l'invention, une solution de maltulose d'une richesse supérieure à 90 % en poids, et d'un taux de matière sèche supérieur à 65 % en poids est amenée par exemple de 33°C à 25°C en moins de 26 heures ou bien encore, une solution de maltulose d'une richesse supérieure à 70 % en poids, et d'un taux de matière sèche supérieur à 50 % en poids est amenée de 25°C à 15°C en moins de 24 heures.

Cette étape de cristallisation du maltulose peut être effectuée de manière continue ou discontinue, par exemple dans des récipients de type cristallisoirs (malaxeurs horizontaux), refroidis par double enveloppe. De préférence, ladite solution aqueuse de maltulose est versée dans un récipient de type cristallisoir, sous agitation, où le refroidissement est assuré par circulation d'eau dans la double paroi.

Enfin, les cristaux de maltulose monohydrate sont ensuite collectés par toute méthode connue en soi par l'homme du métier, par exemple, par essorage ou filtration de la solution de maltulose cristallisé.

De préférence, les cristaux sont ensuite purifiés par clairçage à l'eau ou éventuellement par un peu d'éthanol, puis séchés à une température inférieure au point de fusion du maltulose cristallisé, préférentiellement à une température inférieure à 80°C, par toute méthode en elle-même connue, par exemple en étuve ou sur lit fluidisé.

La mise en oeuvre du procédé conforme à l'invention permet d'obtenir des cristaux d'une taille généralement supérieure à 5 µm, préférentiellement comprise entre 10 µm et 100 µm et d'une richesse supérieure à 90 % et préférentiellement supérieure à 95 %.

D'autres caractéristiques et avantages de l'invention apparaîtront à la lecture des exemples non limitatifs décrits ci-dessous.

### Exemple 1. Cristallisation d'une solution aqueuse de maltulose d'une richesse de 95 % en poids

Une solution de maltose à 87 % est mélangée avec une quantité équimolaire d'acide borique, et le pH de la solution résultante est ajustée à 11 par addition de soude. L'isomérisation du maltose en maltulose est ensuite effectuée par chauffage de la dite solution à 50°C pendant 4 heures. La solution est finalement refroidie à 30°C et son pH ajusté à 2 par ajout d'acide chlorydrique.

A l'issue de cette réaction, le rendement d'isomérisation du maltose en maltulose est de 86 %.

Deux jets de cristallisation assurent l'élimination de 80 % de l'acide borique initial. La solution de maltulose est ensuite purifiée par chromatographie sur résine cationique forte sous forme Na+, dont le support est constitué de divinyl benzylidène à 4 %, suivi d'une étape de déminéralisation.

La solution de maltulose à 95% de richesse, obtenue à l'issue de cette première étape, est concentrée par évaporation sous vide à une température de 60°C, jusqu'à un taux de matière sèche d'environ 67,5 %. Son degré de sursaturation est alors inférieur à 1.

2 litres de cette solution sont refroidis et amenés à une température de 33°C, ce qui conduit à obtenir un degré de sursaturation en maltulose d'une valeur de l'ordre de 1,1.

On favorise alors la nucléation par cisaillement de la solution avec un homogénéiseur de type Ultra-Turrax, commercialisé par la Société BIOBLOCK.

On poursuit alors le refroidissement linéairement jusqu'à 25°C en 26 heures, dans un cristallisoir à double paroi, sous agitation.

En fin de cristallisation, les cristaux sont séparés de la liqueur mère à l'aide d'une essoreuse centrifuge conventionnelle et pesés. Au cours de cette étape, les 277 g de cristaux sont claircés avec 300 ml d'eau puis avec 200 ml d'éthanol.

Les cristaux sont ensuite séchés dans un séchoir à lit fluidisé pendant 15 min à 60°C.

Le rendement de cristallisation est de 15 % en poids exprimé en poids de maltulose cristallisé sur le poids de maltulose départ. La pureté en maltulose des cristaux récupérés est de 97 % sur sec. La teneur en eau est de 5,4 %.

Le point de fusion des cristaux de maltulose monohydrate ainsi obtenus est de 115,5 °C.

### Exemple 2. Cristallisation d'une solution aqueuse de maltulose d'une richesse de 75 % en poids

La cristallisation est effectuée à partir d'une solution de maltulose de 75 % en poids obtenue par l'isomérisation d'une solution aqueuse de maltose telle que décrit dans l'exemple 1 et déminéralisé.

La solution est alors concentrée à un taux de matière sèche de 65 % par évaporation sous vide à une température de 60 °C et refroidie à 25°C pour atteindre un degré de sursaturation en maltulose supérieur à 1.

1 litre de cette solution de maltulose est ensemencé à 25 °C par l'ajoût de 10 grammes de germes de maltulose tel qu'obtenus dans l'exemple 1.

La solution est ensuite refroidie jusquà 15°C en 24 heures. Le rendement de cristallisation est de 12 % en poids. La pureté des cristaux de maltulose monohydrate récupérés est de 95 % sur sec.

## Revendications

1. Procédé de préparation de maltulose monohydrate cristallisé, **caractérisé par le fait qu'**il comporte les étapes suivantes :
- Préparation d'une solution aqueuse de maltulose d'une richesse supérieure à 65% en poids,
- Concentration de la solution aqueuse de maltulose à une matière sèche supérieure à 50% en poids et à une température supérieure à 60°C telle que le degré de sursaturation du maltulose soit inférieur à 1,
- Refroidissement de ladite solution concentrée à une température comprise entre 20° et 50°C, de manière à porter le degré de sursaturation du maltulose à une valeur supérieure à 1,
- Cristallisation du maltulose dans ladite solution sursaturée, en la refroidissant à vitesse contrôlée et sous agitation, et
- Séparation, récupération et séchage des cristaux de maltulose monohydrate ainsi obtenus.

2. Procédé selon la revendication 1, **caractérisée par le fait que** l'on prépare une solution aqueuse de maltulose d'une richesse supérieure à 85% en poids et plus préférentiellement comprise entre 85 et 97% en poids.

3. Procédé selon l'une ou l'autre des revendications 1 et 2, **caractérisé par le fait que** l'on concentre la solution aqueuse de maltulose jusqu'à une matière sèche comprise entre 65 et 75 % en poids.

4. Procédé selon l'une quelconque des revendications 1 à 3, **caractérisé par le fait que** l'on refroidit ladite solution concentrée à une température comprise entre 25°C et 35°C.

5. Procédé selon l'une quelconque des revendications 1 à 4, **caractérisé par le fait que** l'on fait cristalliser le maltulose en provoquant une nucléation spontanée par cisaillement de ladite solution sursaturée.

6. Procédé selon l'une quelconque des revendications 1 à 4, **caractérisé par le fait que** l'on fait cristalliser la maltulose par ensemencement de ladite soplution sursaturée avec des germes de maltulose.

7. Procédé selon l'une quelconque des revendications 1 à 6, **caractérisé par le fait que** l'on refroidit la solution aqueuse sursaturée jusqu'à une température d'au plus égale à 10°C dans un délai d'au moins 15 heures.

8. Procédé selon l'une quelconque des revendications 1 à 7, **caractérisé par le fait que** les cristaux de maltulose monohydrate obtenus sont collectés par centrifugation et séchés à une température comprise entre 50°C et 80°C.

## Claims

1. A process for the preparation of crystallised maltulose monohydrate, **characterised in that** it comprises the following stages:
- the preparation of an aqueous maltulose solution of a molecular abundance ratio above 65% by weight,
- the concentration of the aqueous maltulose solution to a dry matter ratio of more than 50% by weight and at a temperature such that the maltulose supersaturation degree is less than 1,
- the cooling of said concentrated solution to a temperature of between 20 and 50°C, so as to take the maltulose supersaturation degree to a value above 1,
- the crystallisation of the maltulose in said supersaturated solution, by cooling it at a controlled speed and by stirring, and
- the separation, recuperation and drying of the maltulose monohydrate crystals thus obtained.

2. A process according to claim 1, **characterised in that** an aqueous maltulose solution of a molecular abundance ratio above 85% by weight and more preferentially between 85 and 97% by weight is prepared.

3. A process according to one or other of claims 1 and 2, **characterised in that** the aqueous maltulose solution is concentrated to a dry matter of between 65 and 75% by weight.

4. A process according to any one of the claims 1 to 3, **characterised in that** said concentrated solution is cooled to a temperature of between 25°C and 35°C.

5. A process according to any one of the claims 1 to 4, **characterised in that** the maltulose is crystallised by causing spontaneous nucleation by shearing said supersaturated solution.

6. A process according to any one of the claims 1 to 4, **characterised in that** the maltulose is crystallised by seeding said supersaturated solution with maltulose seeds.

7. A process according to any one of the claims 1 to 6, **characterised in that** the supersaturated aqueous solution is cooled to a temperature of at most equal to 10°C within at least 15 hours.

8. A process according to any one of the claims 1 to 7, **characterised in that** the maltulose monohydrate crystals obtained are collected by centrifugation and dried at a temperature of between 50°C and 80°C.

## Patentansprüche

1. Verfahren zur Herstellung von kristallinem Maltulose-Monohydrat, **dadurch gekennzeichnet, daß** es die folgenden Schritte umfaßt:
- Herstellung einer wässrigen Maltuloselösung mit einem Gehalt von mehr als 65 Gew.-%,
- Konzentration der wässrigen Maltuloselösung auf eine Trockenmasse von über 50 Gew.-% und bei einer Temperatur über 60°C, so daß der Übersättigungsgrad der Maltulose kleiner als 1 ist,
- Abkühlung der konzentrierten Lösung auf eine Temperatur zwischen 20° und 50°C, so daß der Übersättigungsgrad der Maltulose auf einen Wert größer als 1 gebracht wird,
- Kristallisation der Maltulose in dieser übersättigten Lösung, indem sie unter Rühren mit gesteuerter Geschwindigkeit gekühlt wird, und
- Abtrennung, Gewinnung und Trocknung der auf diese Weise erhaltenen Maltulose-Monohydrat-Kristalle.

2. Verfahren nach Anspruch 1, **dadurch gekennzeichnet, daß** man eine wässrige Maltuloselösung mit einem Gehalt über 85 Gew.-% und vorzugsweise zwischen 85 und 97 Gew.-% herstellt.

3. Verfahren nach einem der Ansprüche 1 und 2, **dadurch gekennzeichnet, daß** man die wässrige Maltoselösung bis zu einer Trockenmasse zwischen 65 und 75 Gew.-% konzentriert.

4. Verfahren nach einem der Ansprüche 1 bis 3, **dadurch gekennzeichnet, daß** man die konzentrierte Lösung auf eine Temperatur zwischen 25°C und 35°C kühlt.

5. Verfahren nach einem der Ansprüche 1 bis 4, **dadurch gekennzeichnet, daß** man die Maltulose kristallisieren läßt, indem man durch Scheren der übersättigten Lösung eine Selbstnukleation bewirkt.

6. Verfahren nach einem der Ansprüche 1 bis 4, **dadurch gekennzeichnet, daß** man die Maltulose durch Beimpfen der übersättigten Lösung mit Maltulosekeimen kristallisieren läßt.

7. Verfahren nach einem der Ansprüche 1 bis 6, **dadurch gekennzeichnet, daß** man die übersättigte wässrige Lösung bis auf eine Temperatur von höchstens gleich 10°C in einer Zeit von mindestens 15 Stunden kühlt.

8. Verfahren nach einem der Ansprüche 1 bis 7, **dadurch gekennzeichnet, daß** die erhaltenen Maltulose-Monohydrat-Kristalle durch Zentrifugieren gesammelt und bei einer Temperatur zwischen 50°C und 80°C getrocknet werden.
